# EUROPEAN PATENT APPLICATION

(11) **EP 3 907 291 A1**
(43) Date of publication of application: **10.11.2021**
(21) Application number: 21157493.4
(22) Date of filing: 13.06.2007
(51) Int. Cl.: C12P 5/00

(54) **SYSTEM FOR THE PRODUCTION OF METHANE FROM CO2**

(30) Priority: 13.06.2006 US 813020 P
(62) Divisional of application: 07872663.5
(71) Applicant: The University of Chicago, Chicago, IL 60637 (US)
(72) Inventor: Mets, Laurens, Chicago, Illinois 60610 (US)
(74) Representative: D Young & Co LLP

(57) **Abstract**

A method of converting CO₂ gas produced during industrial processes comprising contacting methanogenic archaea with the CO₂ gas under suitable conditions to produce methane.

## Description

### CROSS REFERENCE TO RELATED APPLICATIONS

This application claims priority to United State Provisional Application No. 60/813,020 filed June 13, 2006, which is incorporated by reference in its entirety.

### BACKGROUND OF THE INVENTION

Energy self-sufficiency and sustainable energy systems with lower environmental impacts are critical national goals. Increased use of biomass-derived ethanol as a fuel is advantageous because it uses solar energy, rather than fossil fuel energy, as a portion of its energy input and CO₂ obtained by photosynthesis from the environment as a portion of its material requirement for energy carriers. At present ethanol production from corn requires significant energy input from fossil fuels for distillation of the final product and for drying of fermentation residues for use in animal feed. Present domestic ethanol production methods, therefore, are not energetically or economically competitive with ethanol produced abroad from sugar cane. In addition, one third of the carbon in the corn starch is released as a concentrated CO₂ stream during ethanol production. The U.S. Department of Energy has identified that increasing the energy efficiency and reducing the CO₂ emissions of the fuel ethanol production process is essential for increasing the role of ethanol in meeting our energy needs. Currently, fuel ethanol production relies on federal subsidies for its economic viability. Therefore, it will be important to achieve greater economic efficiency in the ethanol production process if the industry is to be viable and self-sustaining.

The present invention provides a system that reduces the CO₂ emissions from industrial processes, including ethanol production, by using a bioreactor system that uses the emissions to produce methane (natural gas).

### SUMMARY OF THE INVENTION

The present invention provides a system that converts the CO₂ into methane (natural gas). The present invention utilizes CO₂ produced by industrial processes. Examples of processes that that produce CO₂ are biomass fermentation to produce liquid fuels and coal and biomass gasification processes. Gasification is a process that converts carbonaceous materials, such as coal, petroleum, petroleum coke or biomass (living or dead biological material), into carbon monoxide, hydrogen and carbon dioxide. In the system of the present invention, CO₂ industrial waste-gas streams, such as those formed during the production of ethanol or those produced by combined cycle coal fired energy plants, is combined with hydrogen and undergoes a microbial fermentation process catalyzed by methanogenic archaea, producing methane and water. Hydrogen gas may be produced from a variety of sources. In one embodiment, inexpensive electric power can be used to produce hydrogen from water via electrolysis. The integrated electrolysis/methane fermentation system can be viewed as converting an intermittent energy source (e.g. inexpensive off-peak electricity from power plants) to a stable chemical energy store, using hydrogen as an intermediate and methane as the final energy carrier.

The present invention uses a bioreactor containing methanogenic archaea to catalyze the following chemical reaction:

CO₂ + 4H₂ → CH₄ + 2H₂O

This reaction occurs with high efficiency with >95% conversion of CO₂ to methane at moderate temperatures. Suitably the bioreactor conditions will allow a reaction vessel that is 1/10 or less the volume of the ethanol fermentation system to handle all of the CO₂ stream.

In one embodiment, the present invention provides a method of converting carbon dioxide produced during an industrial process to methane comprising contacting a culture comprising methanogenic archaea with H₂ gas and an output gas from an industrial process comprising CO₂ gas in a bioreactor under suitable conditions to produce methane. The industrial process can be coal gasification, biomass gasification, or liquid fuel production by biomass fermentation, suitably ethanol production from a biomass such as corn.

Any suitable methanogenic archaea can be used, and a suitable temperature and pressure for the bioreactor condition can be selected depending at least in part on the methanogenic archaea selected. In some embodiments, suitably pressures within the bioreactor range from about 0.5 atmospheres to about 500 atmospheres. The bioreactor can also contain a source of intermittent agitation of the culture.

The culture conditions should suitably maintaining a redox potential of about -100 mV or less. In one embodiment, this redox potential is maintained by supplying a suitable amount of hydrogen gas.

Also in one embodiment, the methane gas removed from the bioreactor suitably comprises less than about 450 ppm hydrogen sulfide, or alternatively less than about 400 ppm, 300 ppm, 200 ppm or 150 ppm of hydrogen sulfide.

Further, in certain embodiments the industrial output gas at least intermittently further comprises air and/or carbon monoxide. Suitably the industrial output gas comprises about 32% or less air by volume, or between from about 0.1% to about 32% air by volume, or less than about 4% air by volume, or at least about 4%, 8% or 16% air by volume. Suitably the industrial output gas can also comprise less than about 40% carbon monoxide by volume, or less than about 8% carbon monoxide by volume, or at least about 8% or 16% carbon monoxide by volume.

An another embodiment, the bioreactor comprises a culture of methanogenic archaea, a source of an output gas from an industrial process comprising CO₂ that feeds into the bioreactor, a source of hydrogen gas that feeds into the bioreactor, a gas feed from the bioreactor for removing gas from the bioreactor, a feed for providing fresh medium, and a feed for removing the culture.

### DRAWINGS

FIG. 1 shows a design schematic of one embodiment of a CO₂ recapture and methane production plant.
FIG. 2 shows a design schematic of one embodiment of a stratified bioreactor.
FIG. 3 shows a design schematic of a system of bioreactors set up in cascaded serial arrangement.
FIG. 4 is a chart showing the growth curve of methane production of *Methanococcus maripaludis.*
FIG. 5 is a chart demonstrating the effects of agitation of a culture of *Methanococcus maripaludis* with respect to methane production.
FIG. 6 is a chart showing the changes in hydrogen conversion to methane catalyzed by *Methanococcus maripaludis* with respect to changes in the feed rate hydrogen gas into the bioreactor.
FIG. 7 is a chart showing the recovery of methanogenesis in a culture *Methanosarcina barkeri* after exposure to 10 minutes of 100% air.
FIG. 8 is a chart showing the recovery of methanogenesis in a culture *Methanosarcina barkeri* after exposure to 10 minutes of 100% air.
FIG. 9 is a chart showing the recovery of methanogenesis in a culture *Methanosarcina barkeri* after exposure to 90 minutes of 100% air.
FIG. 10 is a chart showing the recovery of methanogenesis in a culture *Methanosarcina barkeri* after exposure to 15 hours of 100% air.
FIG. 11 is a chart showing the recovery of methanogenesis in a culture *Methanococcus maripaludis* after exposure to 10 minutes of 100% air.
FIG. 12 is a chart showing the recovery of methanogenesis in a culture *Methanococcus maripaludis* during exposure of a mixture of air and hydrogen gas.
FIG. 13 is a chart showing the recovery of methanogenesis in a culture *Methanococcus maripaludis* during exposure of a mixture of carbon monoxide and hydrogen gas.
FIG. 14 is a chart showing the recovery of methanogenesis in a culture *Methanococcus maripaludis* during exposure of a mixture of carbon monoxide and hydrogen gas.

### DETAILED DESCRIPTION OF THE INVENTION

The present invention comprises a bioreactor system that can be integrated with industrial processes that produce CO₂ gas as a byproduct. In one embodiment such a process is the production of ethanol from biomass. The invention comprises a bioreactor containing a microbial culture capable of hydrogenotrophic methanogenesis (i.e. the conversion of CO₂ gas plus hydrogen gas to methane gas). The bioreactor is coupled to a hydrogen source and a CO₂ gas source. Suitably the CO₂ gas source is the CO₂ gas stream that is emitted by the production of ethanol. The hydrogen source is suitably hydrogen produced by the electrolysis of water. Suitably this hydrolysis is powered by electricity used in off peak times. The methane produced by the system can be fed back into the ethanol production facility to power various processes, and/or can be stored and sold as fuel.

Microbial cultures suitable for practice of the invention are readily obtainable from public collections of organisms or can be isolated from a variety of environmental sources. Such environmental sources include anaerobic soils and sands, bogs, swamps, marshes, estuaries, dense algal mats, both terrestrial and marine mud and sediments, deep ocean and deep well sites, sewage and organic waste sites and treatment facilities, and animal intestinal tracts and feces. Many pure cultures of single species are suitable. Classified pure cultures are all members of the Archaeal domain [Woese et al. Proc Natl Acad Sci USA 87:4576-4579 (1990) "Towards a natural system of organisms: Proposal for the domains Archaea, Bacteria, and Eucharya.", incorporated herein by reference] and fall within 4 different classes of the Euryarchaea kingdom. Examples of suitable organisms have been classified into 4 different genera within the Methanobacteria class (e.g. *Methanobacterium alcaliphilum*, *Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arboriphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanothermobacter marburgensis, Methanothermobacter thermautotrophicum, Methanothermobacter thermoflexus, Methanothermobacter thermophilus, Methanothermobacter wolfeii, Methanothermus sociabilis),* 5 different genera within the Methanomicrobia class (e.g. *Methanocorpusculum bavaricum, Methanocorpusculum parvum, Methanoculleus chikuoensis, Methanoculleus submarinus, Methanogenium frigidum, Methanogenium liminatans, Methanogenium marinum, Methanosarcina acetivorans, Methanosarcina barkeri, Methanosarcina mazei, Methanosarcina thermophila, Methanomicrobium mobile),* 3 different genera within the Methanococci class (e.g. *Methanocaldococcus jannaschii, Methanococcus aeolicus, Methanococcus maripaludis, Methanococcus vannielii, Methanococcus voltaei, Methanothermococcus thermolithotrophicus),* and one genus within the Methanopyri class (e.g. *Methanopyrus kandleri*)*.* Suitable cultures are available from public culture collections (e.g. the American Type Culture Collection, the Deutsche Sammlung von Mikroorganismen und Zellkulturen GmbH, and the Oregon Collection of Methanogens). Many suitable hydrogenotrophic methanogens, isolated in pure culture and available in public culture collections, have not yet been fully classified. Preferred pure culture organisms include *Methanosarcinia barkeri, Methanococcus maripaludis,* and *Methanothermobacter thermoautotrophicus.*

Suitable cultures of mixtures of two or more microbes are also readily isolated from the specified environmental sources [Bryant et al. Archiv Microbiol 59:20-31 (1967) "Methanobacillus omelianskii, a symbiotic association of two species of bacteria.", incorporated herein by reference]. Suitable mixtures may be consortia in which cells of two or more species are physically associated or they may be syntrophic mixtures in which two or more species cooperate metabolically without physical association. Mixed cultures may have useful properties beyond those available from pure cultures of known hydrogenotrophic methanogens. These properties may include, for instance, resistance to contaminants in the gas feed stream, such as oxygen, ethanol or other trace components, or aggregated growth, which may increase the culture density and volumetric gas processing capacity of the culture.

Suitable cultures of mixed organisms may also be obtained by combining cultures isolated from two or more sources. One or more of the species in a suitable mixed culture should be an Archaeal methanogen. Any non-Archael species may be bacterial or eukaryotic.

Suitable cultures may also be obtained by genetic modification of nonmethanogenic organisms in which genes essential for supporting hydrogenotrophic methanogenesis are transferred from a methanogenic microbe or from a combination of microbes that may or may not be methanogenic on their own. Suitable genetic modification may also be obtained by enzymatic or chemical synthesis of the necessary genes.

The bioreactor system may provide continuous or discontinuous methane production using a continuous hydrogenotrophic methanogenic culture operating under stable conditions. An example of such suitable conditions is set forth below in the examples and is also provided in Schill, N., van Gulik, M., Voisard, D., & von Stockar, U. (1996) Biotechnol & Bioeng 51:645-658. "Continuous cultures limited by a gaseous substrate: development of a simple, unstructured mathematical model and experimental verification with Methanobacterium thermoautotrophicum", incorporated herein by reference. Culture media may be comprised of dilute mineral salts, and should be adapted to the particular culture in use.

The medium should be replenished at a rate suitable to maintain a useful concentration of essential minerals and to eliminate any metabolic products that may inhibit methanogenesis. Dilution rates below 0.1 culture volume per hour are suitable, since they yield high volumetric concentrations of active methane generation capacity. Surprisingly, dilution rates of less than 0.001 volumes was found to provide active methane generating capacity.

Total gas delivery rates (CO₂ plus H₂) in the range of 0.2 to 1 volume of gas (STP) per volume of culture per minute are suitable, since they both maintain and exploit high volumetric concentrations of active methane generation capacity.

In one embodiment, the redox potential is maintained below -100mV or lower during methanogenesis. The method of the present invention encompasses conditions in which the redox potential is transiently increased to above -100 MV, as for example when air is added to the system.

In the examples below the temperature of the culture was maintained near the optimum for growth of the organism used in the culture (e.g. about 35°C to about 37°C for mesophilic organisms such as *Methanosarcinia barkeri* and *Methanococcus maripaludis* or about 60°-65°C for thermophiles such as *Methanothermobacter thermoautotrophicus,* and about 85°C-90°C for organisms such as *Methanocaldococcus jannaschii*)*.* However, it is envisioned that temperatures above or below the temperatures for optimal growth may be used. In fact, higher conversion rates of methane may be obtained at temperatures above the optimal growth rate temperature.

In one embodiment of the invention, a reducing agent is introduced into the fermentation process along with CO₂ and hydrogen, this reducing agent can suitably be hydrogen sulfide or sodium sulfide. In one embodiment, a 4:1 mixture of H₂ and CO₂ gases can be provided at a total gassing rate (vvm) of from 0.1 L gas per L culture per minute [L/(L-min)] to >1.0 L/(L-min), with greater than 95% of the CO₂ converted to methane and the rest of the CO₂ in the input being converted to cellular biomass.

In another embodiment, hydrogen itself can be used as a reductant to maintain the redox potential of the culture in the range (<-100mV) necessary for optimum performance of hydrogenotrophic methanogenesis. Generally, hydrogen gas is provide in the method in concentrations effective in allowing for at least a portion of the carbon dioxide in the bioreactor to be converted into methane.

In another embodiment, the redox potential of the culture can be maintained at <-100mV via an electrochemical cell immersed in the medium.

In another embodiment, the system comprises various methods and/or features that reduce the presence of oxygen in the CO₂ stream that is fed into the bioreactor. When obligate anaerobic methanogenic microorganisms are used to catalyze methane formation, the presence of oxygen may be detrimental to the performance of the process and contaminates the product gas. Therefore the reduction of the presence of oxygen in the CO₂ stream is helpful for improving the process. In one embodiment, the oxygen level is reduced prior to entry of the gas into the fermentation vessel by passing the mixed H₂/CO₂ stream over a palladium catalyst, which converts any trace oxygen to water. In this embodiment, H₂ is provided in an amount above the amount needed in the culture by a 2:1 ratio relative to the contaminating oxygen. In another embodiment, the oxygen is removed by pretreatment of the gas stream in a bioreactor. In this embodiment, the reductant may be provided either by provision of a source of organic material (e.g. glucose, starch, cellulose, fermentation residue from an ethanol plant, whey residue, etc.) that can serve as substrate for an oxidative fermentation. The microbial biological catalyst is chosen to oxidatively ferment the chosen organic source, yielding CO₂ from the contaminant oxygen. In this embodiment, additional H₂ would be provided to enable conversion in the anaerobic fermentor of this additional CO₂ to methane. In another embodiment, oxygen removal is accomplished in the main fermentation vessel via a mixed culture of microbes that includes one capable of oxidative fermentation of an added organic source in addition to the hydrogenotrophic methanogen necessary for methane production. An example of a suitable mixed culture was originally isolated as *"Methanobacillus omelianskii"* and is readily obtained from environmental sources [Bryant et al. Archiv Microbiol 59:20-31 (1967) "Methanobacillus omelianskii, a symbiotic association of two species of bacteria.", incorporated herein by reference]. In another embodiment, an oxygen tolerant methanogen is used in the bioreactor to improve the stability of the methane formation process in the presence of contaminating oxygen. Both *Methanosarcinia barkeri* and *Methanococcus maripaludis* are sufficiently oxygen tolerant in the presence of contaminating oxygen.

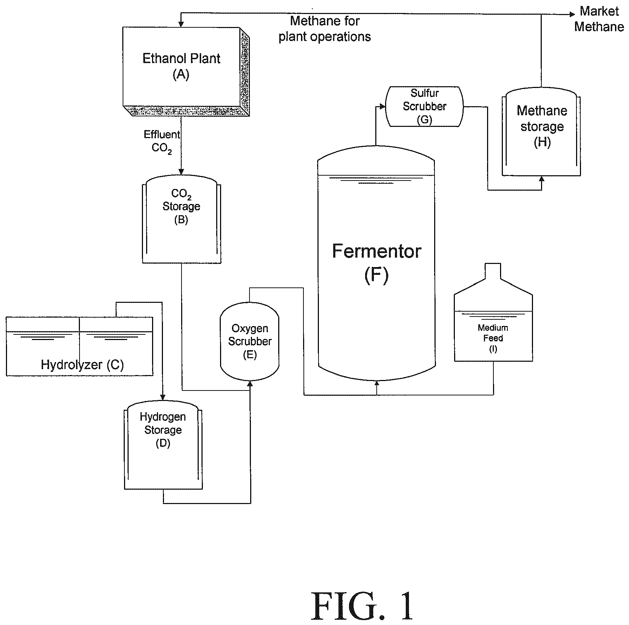

FIG. 1 depicts one embodiment of a CO₂ recapture and methane production plant using the methods set forth above. An industrial CO₂ source (A)-e.g. fuel ethanol plant - with CO₂ effluent and natural gas demand, vents CO₂ to a CO₂ collection and storage tank (B). A hydrolyzer (C) produces hydrogen, suitably from electrolysis. Hydrogen produce by the hydrolyzer (c) is collected in a hydrogen storage tank (D). The hydrogen and CO₂ from their respective storage tanks are fed through an oxygen scrubber (E) for removal of oxygen from the CO₂ effluent stream. After passing through the oxygen scrubber (E), the hydrogen and CO₂ are feed into a fermentor/bioreactor system (F) for conversion of CO₂ + H₂ to methane. A storage tank providing medium (I) is also connected to the fermentor/bioreactor system (F) to provide for replenishment of nutrients in the fermentor. The methane gas vented from the fermentor/bioreactor (F) passes through a sulfur scrubber (G) for recovering sulfur from the product methane stream. The methane gas can then be stored in a methane storage tank (H).

A bioreactor, also known as a fermentor vessel, as set forth in the invention is any suitable vessel in which methanogenesis can take place. Suitable bioreactors to be used in the present invention should be sized relative to the volume of the CO₂ source. Typical streams of 2,200,000 lb CO₂/day from a 100,000,000 gal/yr ethanol plant would require a CO₂ recovery/methane production fermentor of about 750,000 gal total capacity. Fermentor vessels similar to the 750,000 gal individual fermentor units installed in such an ethanol plant would be suitable.

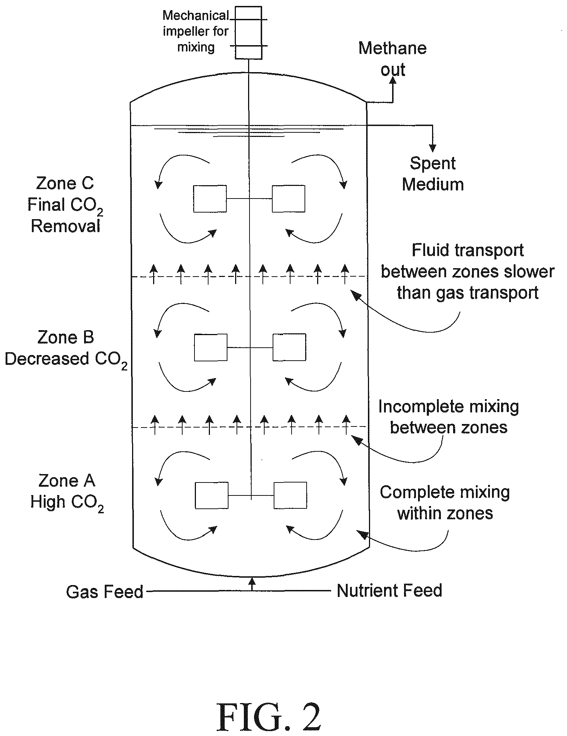

FIG. 2 depicts one embodiment of a stratified bioreactor that can be used in the present invention. In this embodiment, the bioreactor has the CO₂ and hydrogen entering into the bottom of the bioreactor along with the nutrients for the bioreactor. A mechanical impeller is positioned on the top of the bioreactor and is used to move a mixing apparatus within the bioreactor. The bioreactor has three zones, A, B and C. Zone A at the bottom of the reactor is a high CO₂ zone. Zone B, in the middle of the bioreactor has a decreased CO₂ presence, and Zone C at the top end of the reactor has little if any CO₂. The methane produced, and the spent medium is removed from the top of the bioreactor.

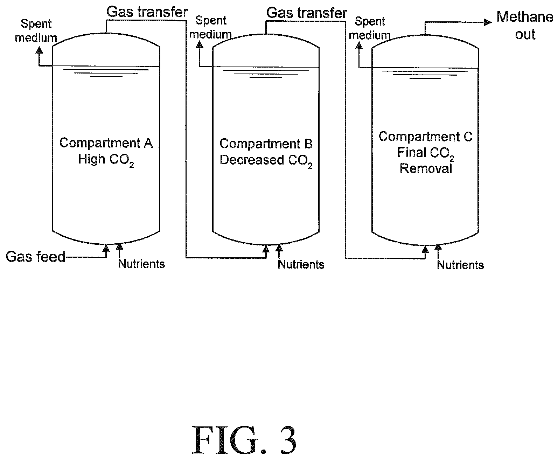

FIG. 3 depicts one embodiment of a cascaded bioreactor that can be used in the present invention. In this embodiment, the hydrogen, CO₂ and cell nutrients are fed into the bottom of a first compartment (A). In this compartment (A), even after fermentation, there is still a high level of CO₂. The gas produced by the fermentation reaction in the first compartment (A) is then transferred from the top of the first compartment to the bottom of a second compartment (B) along with cell nutrients. In this second compartment (B) the CO₂ level is decreased from the levels found in the first compartment (A). The gas produced by the fermentation reaction in the second compartment (B) is transferred from the top of the second compartment (B) to the bottom of a third compartment (C) along with cell nutrients. In this third compartment (C) most if not all of the CO₂ has been removed and only the methane gas is left to be removed from the top of the compartment. In each of the compartments, spent medium can be removed from the compartments.

### Example 1 - General Setup for Bench Scale Bioreactor

A bench-scale bioreactor was used to test a series of variables important in the design and operation of an industrial scale bioreactor. A 1.3L fermenter vessel (bioreactor) (BioFlo 110, New Brunswick), fitted with an Ingold autoclavable pH electrode for measuring pH in the medium and a Lazar Labs double junction platinum band autoclavable ORP electrode for measuring the oxidation-reduction potential (ORP) of the medium was used in the following experiments. The bioreactor contained 1 L culture medium and was stirred at 400rpm with a Rushton impeller. With 1L of medium, the bioreactor has a headspace of 300cc of gas. The chamber was also fitted with a peristaltic pump that could control the addition of a chemical reductant, such as Na₂S. A second peristaltic pump controlled the constant addition of fresh culture medium to the vessel to enable continuous culture operation. A third peristaltic pump was used to remove excess liquid from the culture vessel, maintaining a constant volume of 1 L. The excess liquid included the metabolic water generated during methanogenesis as well as increased medium volume from continuous culture operation. The temperature of the culture was controlled by a heating blanket. Gas mixtures were introduced via a sparger at the bottom of the vessel. The composition of the gas mixture was controlled by three mass flow controllers, one for H₂, one for CO₂, and a third that could be used for controlling addition of air, CO, or N₂. Generally, a gas composition of 1 volume CO₂ to 4 volumes of H₂ was used and was passed over a palladium catalyst (Alfa AESAR) prior to introduction to the culture. The culture in the bioreactor was maintained at about 1 atmosphere of pressure. The gas exiting the culture vessel at ambient atmospheric pressure was passed through a condenser at 4°C to reduce water vapor content. The composition of the effluent gas stream was analyzed by a Cirrus quadrupole mass spectrometer continually scanning the mass range of 1 to 50 atomic mass units. To correct for variations in ambient pressure over time, each scan was normalized to the sum of detected masses. Composition of individual gasses was determined by comparison with mixtures of various composition generated with the mass flow control system. Measurements were made of the amount of methane produced by a given volume of culture per unit time, as well as the efficiency of conversion of input CO₂ and H₂ to methane.

### Example 2 - Bench Scale Bioreactor using Methanococcus maripaludis

The general setup of Example 1 was used with the organism *Methanococcus maripaludis. Methanococcus maripaludis* is grown at 37°C in modified McCas medium containing the following components per L of medium: KCl 0.335g, MgCl₂•6H₂O 2.75g, MgSO₄•7H2O 3.45g, CaCl₂•2H₂O 0.14g, NH₄Cl 0.5g, NaHCO₃ 8.4g, NaCl 22g, K₂HPO₄ 0.14g, FeSO₄•7H₂O 9.5mg, Resazurin 1mg, Casamino acids 2g, cysteine•H₂O•HCl 0.5g, Na₃Citrate•2H₂O 21mg, MnSO₄•2H₂O 5mg, CoCl₂(•6H₂O) 1mg, ZnSO₄(•7H2O) 1mg, CuSO₄•5H₂O 0.1mg, AlK(SO₄)₂ 0.1mg, H₃BO₄ 0.1mg, Na₂MoO₄•2H₂O 1mg, NiCl₂•6H₂O 0.25mg, Na₂SeO₃ 2mg, V(III)Cl 0.1mg, Na₂WO₄•2H₂O 1mg, biotin 0.02mg, folic acid 0.02mg, pyridoxine HCl 0.10mg, thiamine HCl 0.05mg, riboflavin 0.05mg, nicotinic acid 0.05mg, DL-calcium pantothenate 0.05mg, vitamin B12 0.001mg, p-aminobenzoic acid 0.05mg, lipoic acid 0.05mg. After autoclaving and before inoculation, the medium was reduced by the addition of 0.5g/L Na₂S from a 50x anaerobic, sterile stock solution, yielding an ORP of the medium below -100mV. The medium was equilibrated prior to inoculation with a gas phase containing 0.2 atmosphere partial pressure of CO₂ to yield a pH in the range of 7.2-7.3. The initial medium used to start the culture contained, in addition to the above components, 1.4g/L NaAcetate•3H₂O, but the medium reservoir used in continuous culture conditions lacked the addition of acetate.

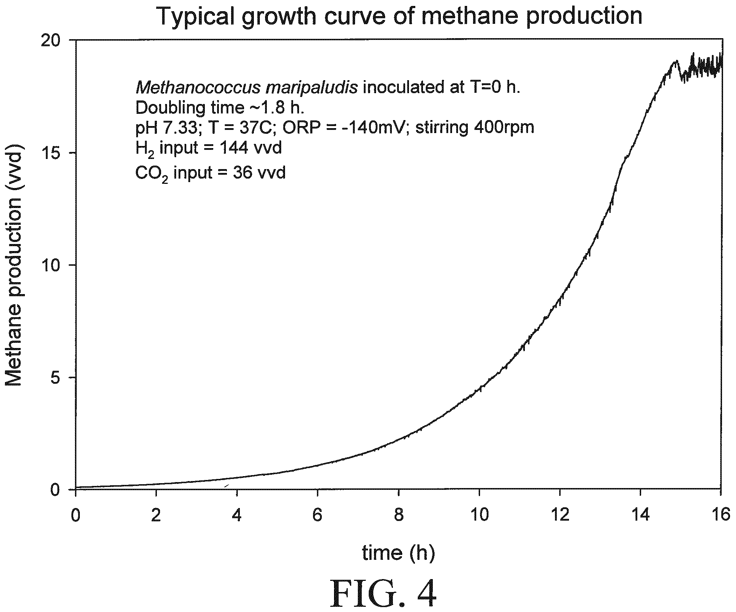

1 L of the fresh medium was initially inoculated with 5 mL of *Methanococcus maripaludis* in a stationary phase, and methane production was monitored over time. As shown in FIG. 4, a gas feed of 4:1 H₂:CO₂ (125cc/min or 180 volumes of gas (STP) per volume of culture per day (VVD), 144 vvd H₂ and 36 vvd CO₂) was provided at a culture of pH 7.33 and an ORP of -140mV. During the growth phase, the rate of methane production is limited by the available biological catalysts for the reaction. The methane production rate stabilized once the dissolved hydrogen in the medium was depleted. The stabilized rate is limited by the physical process of gas-to-liquid mass transfer of hydrogen, rather than by biological factors. Once this transition to stable methane production was observed, the culture was switched to continuous culture conditions in which fresh medium was added at a constant rate of 0.94 ml/h, or 22.5ml/day. It was found that a slower input of fresh culture medium led to a denser culture and hence better volumetric performance. At the limit of no fresh medium, however, it was found that the culture ultimately dies.

### Example 3 - Effect of agitation on methane production of Methanococcus maripaludis

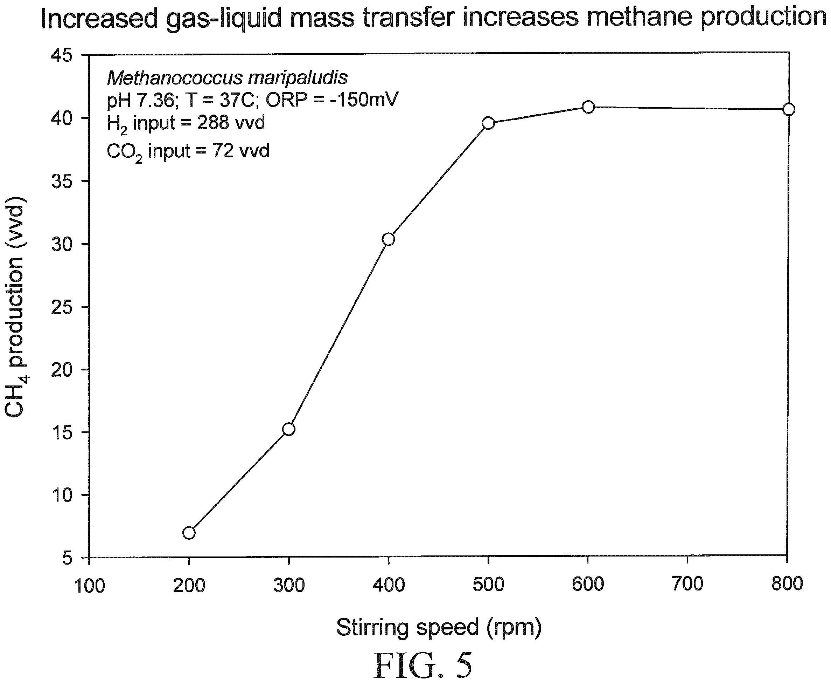

The turbidity of the culture obtained in Example 2 continued to increase after the gas-to-liquid mass transfer-limited rate of methane production was reached, providing an excess of biological catalytic capacity. This additional catalytic capacity can be accessed by changing physical parameters that increase the gas-to-liquid mass transfer rate. As shown in FIG. 5, the mixing rate in the culture was varied from the standard 400rpm. A total feed rate of a 4:1 H₂:CO₂ gas mixture ( 250cc/min (288 vvd H₂; 72 vvd CO₂)) was used. At this gas feed rate, the conversion efficiency of both CO₂ and hydrogen reaches 55-56% at higher mixing speeds demonstrating that higher stirring rates increases the gas-to-liquid mass transfer and therefore higher methane production. Other abiotic methods that may be used to increase the gas-to-liquid mass transfer and hence the methane production rate include 1) increased gas pressure and 2) increased temperature. Some methanognic archaea can thrive at pressures over 500 atmospheres. With respect to different temperature conditions, thermophilic methanogens, such as *Methanothermobacter thermoautotrophicus* (at about 60°C-65°C) or *Methanocaldococcus jannaschii* (at about 85°-90°C), can be used as the biological catalyst.

### Example 4 - Conversion Efficiency of H₂ by Methanococcus maripaludis

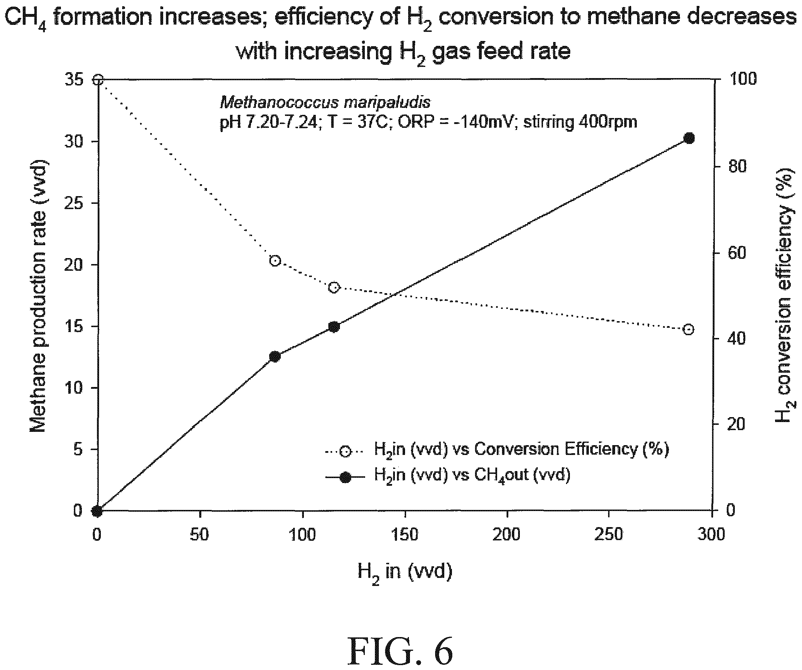

A culture of *Methanococcus maripaludis* was setup in a bioreactor as set forth in Example 2. As shown in FIG. 6, gas was fed to a mature culture at varying rates, maintaining a 4:1 hydrogen to carbon dioxide ratio. Once the culture was above the gas-liquid mass transfer-limited cell density, it was found that methane production could be increased by increasing the H₂ gas feed rate. However, at higher H₂ gas feed rates, it was found that a decreasing proportion of the H₂ gas was converted to methane. The converse was also found to be true, that at lower gas feed rates, hydrogen was converted more efficiently to methane. Because the volume of feed gas (4 volumes of hydrogen plus 1 volume of CO₂) decreases as it is converted to methane (1 volume of methane product), a cascade or stratified bioreactor system as shown in FIG. 2 and FIG. 3 is advantageous. In a serial bioreactor system as shown in FIG. 3, the residual hydrogen in the effluent gas from a first fermenter would provide a lower feed rate to a second fermenter and would therefore be converted at higher efficiency in the second fermenter. This phenomenon can be used to obtain a highly efficient conversion in a cascaded fermenter design

### Example 5 - Bench Scale Bioreactor using Methanosarcina barkeri

The general setup of Example 1 was used with the organism *Methanosarcina barkeri. Methanosarcina barkeri* (strain DSM 804 obtained from the Deutsche Sammlung von Mikroorganismen und Zellkulturen GmbH) was grown at 35°C in MS enriched medium containing the following components per L of medium: NaHCO₃ 8.4g, yeast extract 2.0g, trypticase peptones 2.0g, mercaptoethanesulfonic acid 0.5g, NH₄Cl 1.0g, K₂HPO₄•7H2O 0.4g, MgCl₂•7H₂O 1.0g, CaCl₂•2H₂O 0.4g, Resazurin 1mg, cysteine•H₂O•HCl 0.25g, Na₂EDTA•2H₂O 5mg, MnCl₂•4H₂O 1mg, CoCl₂(•6H₂O) 1.5mg, FeSO₄•7H₂O 1mg, ZnCl₂ 1mg, AlCl₃•6H₂O 0.4mg, Na₂WO₄•2H₂O 0.3mg, CuCl 0.2mg, NiSO₄•6H₂O 0.2mg, H₂SeO₃ 0.1mg, H₃BO₄ 0.1mg, Na₂MoO₄•2H₂O 0.1mg, biotin 0.02mg, folic acid 0.02mg, pyridoxine HCl 0.10mg, thiamine HCl 0.05mg, riboflavin 0.05mg, nicotinic acid 0.05mg, DL-calcium pantothenate 0.05mg, vitamin B12 0.001mg, p-aminobenzoic acid 0.05mg, lipoic acid 0.05mg. After autoclaving and before inoculation, the medium was reduced by the addition of 0.5g/L Na₂S from a 50x anaerobic, sterile stock solution, yielding an ORP of the medium below -100mV. The medium was equilibrated prior to inoculation with a gas phase containing 0.2 atmosphere partial pressure of CO₂ to yield a pH in the range of 6.8-7.0.

1 L of the fresh medium was initially inoculated with 20 mL *Methanosarcina barkeri* in a stationary phase, and methane production was monitored over time. Once the transition to stable methane production was observed, the culture was switched to continuous culture conditions in which fresh medium was added at a constant rate of 0.94 ml/h, or 22.5ml/day. It was found that a slower input of fresh culture medium led to a denser culture and hence better volumetric performance. At the limit of no fresh medium, however, it was found that the culture ultimately dies.

Example 6 - Recovery from Oxygen Exposure - Recovery of *Methanosarcina barkeri* with exposure of 10 minutes of air.

Methanogenic organisms are regarded as extremely strict anaerobes. Oxygen is known as an inhibitor of the enzyme catalysts of both hydrogen uptake and methanogenesis. A low oxidation-reduction potential (ORP) in the growth medium is regarded as important to methanogenesis. Air is a possible contaminant of carbon dioxide streams that could be used to support energy storage in the form of methane and so the effects of air on the capacity of the cultures to produce methane was examined.

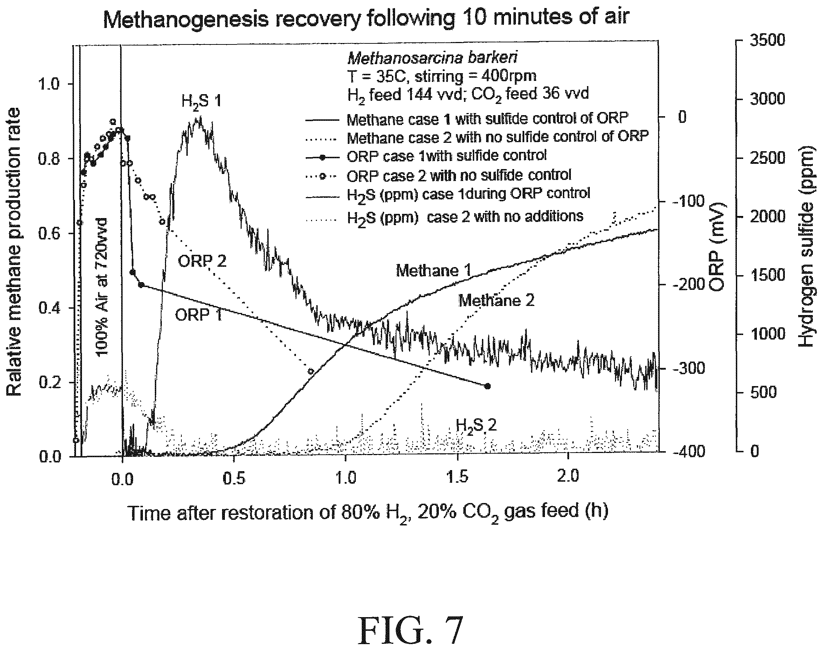

FIG. 7 shows the recovery of the methanogenic activity of *Methanosarcina barkeri* after exposure to air. A bench bioreactor containing *Methanosarcina barkeri* was prepared as set forth in Example 5. Two experiments were performed involving exposing the culture to 100% air for 10 minutes at a flow rate of 500cc/min. Ambient air comprises approximately (by molar content/volume) 78% nitrogen, 21% oxygen, 1% argon, 0.04% carbon dioxide, trace amounts of other gases, and a variable amount (average around 1%) of water vapor. During exposure to 100% air, methanogenesis stopped and the ORP of the culture medium rose. The air used in the experiment also displaces CO₂ dissolved in the medium, causing the pH to rise (not shown in this figure). Following the 10 minute exposure to 100% air, gas flows of H₂ and CO₂ were restored (100cc/min H₂, 25cc/min CO₂).

In the first experiment, 1.5ml of a 2.5% solution of sulfide (Na₂S•7H₂O ) was added within 4 minutes of terminating air feed and restoring the H₂/CO₂ gas feed. Sulfide is widely used to control the ORP of the cultures, control that is regarded as essential. In another experiment, so sulfide was added. The dotted curves show a case in which no sulfide was added and the solid line shows the recovery of the culture where sulfide was added. In both cases, methanogenesis recovers. The figure shows that addition of sulfide for ORP control in case 1 causes the emitted hydrogen sulfide to rise to 3000ppm. In the case of ORP control with hydrogen (no sulfide addition), the hydrogen sulfide level is at or below the limit of detection of the mass spectrometer under these operating conditions (50-100 ppm). Methanogenesis begins to recover more quickly in the case of ORP control with sulfide, but the experiment shows that sulfide is not essential for recovery. The presence of the hydrogen in the gas phase is sufficient to reduce the ORP of the culture to enable methanogenesis, no additional control of the ORP of the culture is required. The lack of necessity of sulfide is of note in that methanogenic cultures are typically maintained at 10,000 ppm hydrogen sulfide in the gas phase. Such high levels of sulfide are not tolerated in certain industrial process, for instance, natural gas pipeline tariffs in the United States set maximum levels of hydrogen sulfide content of natural gas ranging from 4-16ppm, depending upon the pipeline system.

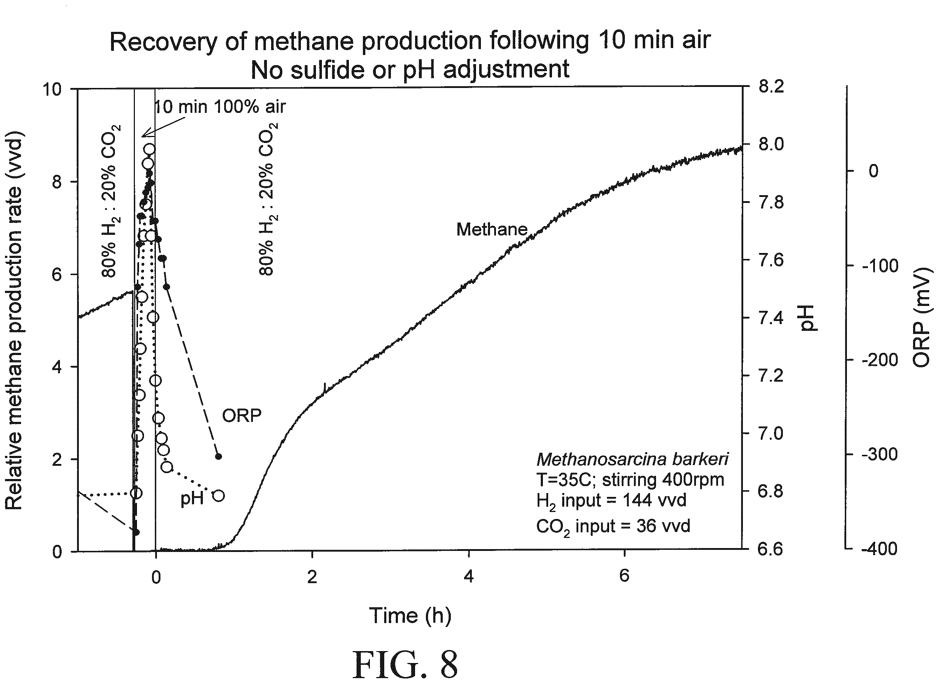

FIG. 8 also shows the recovery of *Methanosarcina barkeri* after the air exposure above (absent the addition of sulfide) after 7 hours.

### Example 7 - Recovery from Oxygen Exposure - Recovery of Methanosarcina barkeri with exposure of 90 minutes of air.

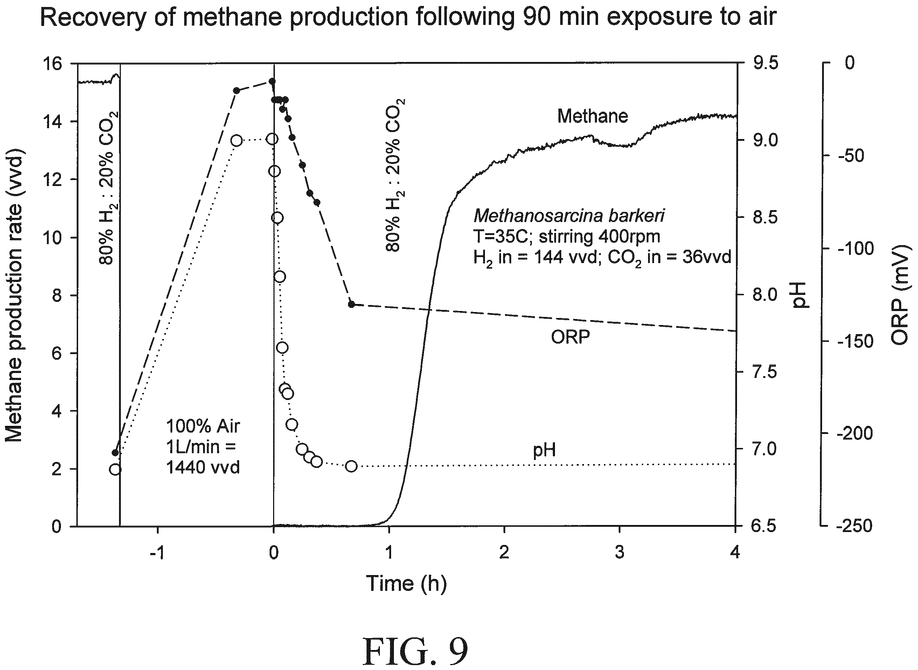

FIG. 9 shows the recovery of *Methanosarcina barkeri* after 15 hours of exposure to air. A bench bioreactor containing *Methanosarcina barkeri* was prepared as set forth in Example 5. The culture was exposed to 100% air for 90 minutes introduced at 1L/min (1440 vvd). During exposure to 100% air, methanogenesis stopped and the ORP of the culture medium rose. The air used in the experiment also displaced CO₂ dissolved in the medium, causing the pH to rise. Following the 90 minute exposure to 100% air, gas flows of H₂ and CO₂ were restored (100cc/min H₂ (144 vvd) and 25cc/min CO₂ (36 vvd). Restoration of hydrogen as a reductant was sufficient to reduce the ORP to levels that favor methanogenesis. Methane production began within 1 hr of restoring 4:1 H₂:CO₂ gas phase (100cc/min H₂, 25cc/min CO₂). Full recovery of methane production was achieved within 3-4 hrs.

### Example 8 - Recovery from Oxygen Exposure - Recovery of Methanosarcina barkeri with exposure of 15 hours of air.

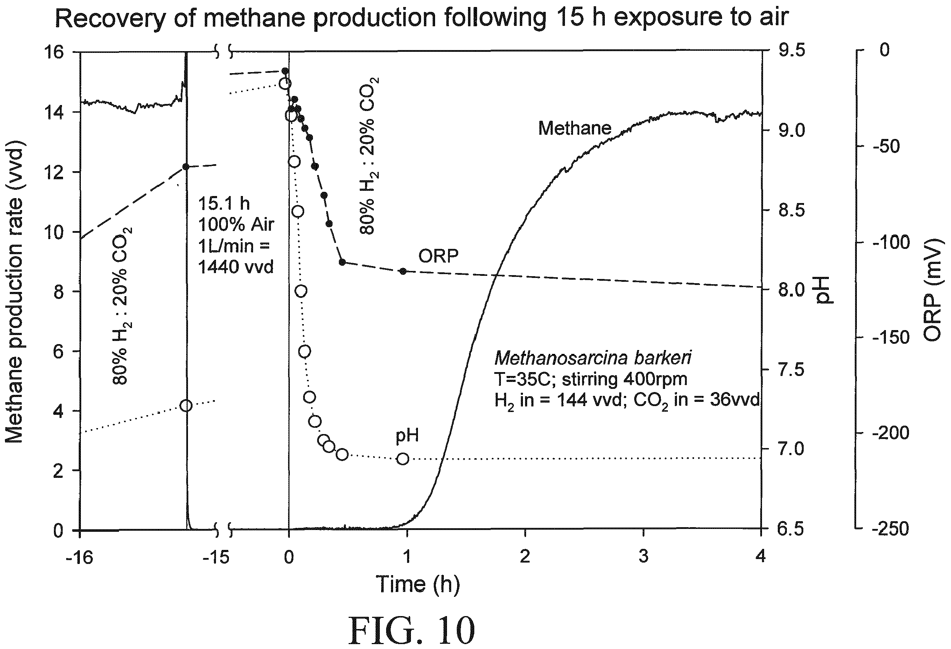

FIG. 10 shows the recovery of *Methanosarcina barkeri* after 15 hours of exposure to air. A bench bioreactor containing *Methanosarcina barkeri* was prepared as set forth in Example 5. The culture was exposed to 100% air for 15.1 hours at a flow rate of 1L/min. During exposure to 100% air, methanogenesis stopped and the ORP of the culture medium rose to about -10 mV. The air used in the experiment also displaced CO₂ dissolved in the medium, causing the pH to rise to 9.3. Following the 15.1 hour exposure to 100% air, gas flows of H₂ and CO₂ were restored (100cc/min H₂, 25cc/min CO₂). Restoration of hydrogen as a reductant was sufficient to reduce the ORP to levels that favor methanogenesis. Methane production began within 1.1 hr of restoring 4:1 H₂:CO₂ gas phase (100cc/min H₂, 25cc/min CO₂). Full recovery of methane production was achieved within 3 hrs.

### Example 9 - Recovery from Oxygen Exposure - Recovery of Methanococcus maripaludis with exposure of 10 minutes of air.

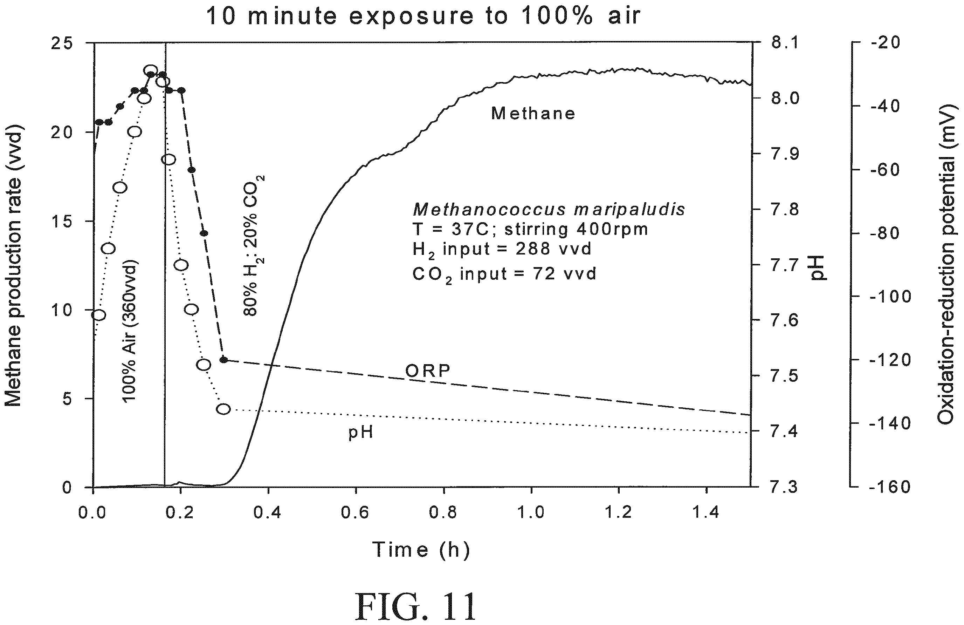

FIG. 11 shows the recovery of the methanogenic activity of *Methanococcus maripaludis* after exposure to air. A bench bioreactor containing *Methanococcus maripaludis* was prepared as set forth in Example 2. The culture was exposed to 100% air for 10 minutes at 360vvd. During exposure to 100% air, methanogenesis stopped and the ORP of the culture medium rose. The air used in the experiment also displaces CO₂ dissolved in the medium, causing the pH to rise. Following the 10 minute exposure to 100% air, gas flows of H₂ and CO₂ were restored (288vvd H₂, 72vvd CO₂). Methanogenesis in was shown to have recovered within 10 min, with full recovery of methane production rate occurring within 1.5 hours.

### Example 10 - Maintained methane production by Methanococcus maripaludis in the presence of air.

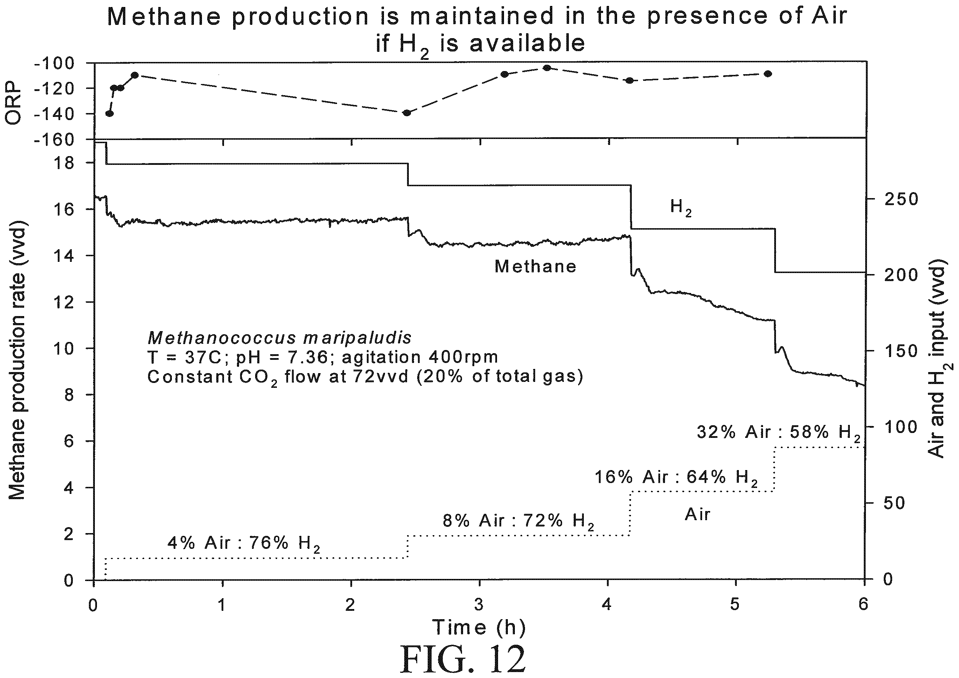

FIG. 12 shows that methane production can continue even in the presence of air provided that hydrogen is present to maintain the ORP of the culture at productive levels. A bench bioreactor containing *Methanococcus maripaludis* was prepared as set forth in Example 2. The culture was exposed to a mixture of 4% air and 76% hydrogen at a flow rate of 100cc/min and CO₂ at 25cc/min for a period of 2.3 hours. The percentage of air was then increased to 8%, providing a mixture of 8% air and 72% hydrogen at a flow rate of 100cc/min and CO₂ at 25cc/min for a period of 1.7 hours. The percentage of air was then increased to 16%, providing a mixture of 16% air and 64% hydrogen at a flow rate of 100cc/min and CO₂ at 25cc/min for a period of 1.1 hours. Finally, the percentage of air was then increased to 32%, providing a mixture of 32% air and 58% hydrogen at a flow rate of 100cc/min and CO₂ at 25cc/min for a period of 0.6 hours. Methane production continues even in the presence of air provided that hydrogen is present to maintain the ORP of the culture at productive levels. Up to 4% air (0.8% oxygen) is tolerated without a persistent reduction in methane production. The efficiency of conversion of the input hydrogen to methane remains unaffected at 22-23% under the conditions of the experiment until the air concentration rises from 8% to 16% of the total gas mix.

Note that the gas mixtures produced by the culture under these conditions could be explosive, since the oxygen is not consumed by the organisms and appears in the effluent gas stream. A potentiostat culture system provides a method for maintaining the ORP of the culture during air exposure without introducing hydrogen or generating methane.

### Example 11 - Recovery from carbon monoxide exposure by Methanococcus maripaludis.

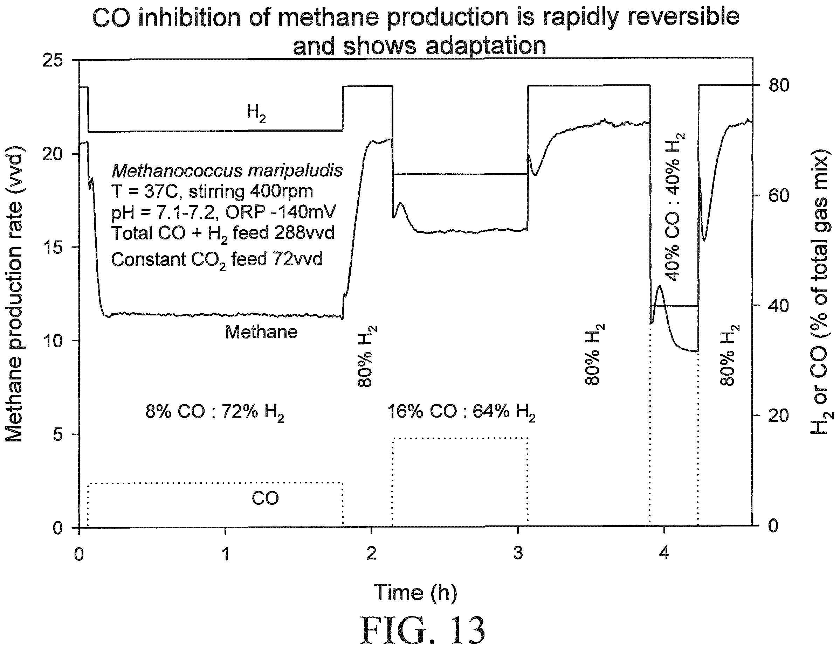

Carbon monoxide is another known inhibitor of enzymes involved in both hydrogen uptake and methanogenesis. CO is a potential contaminant of CO₂ and hydrogen streams derived from gasification of coal or biomass resources. The effect CO on methane formation by methanogen cultures was examined. FIG. 13 shows the recovery of the methanogenic activity of *Methanococcus maripaludis* after exposure to carbon monoxide. A bench bioreactor containing *Methanococcus maripaludis* was prepared as set forth in Example 2. In this experiment, the pH of the culture was maintained constant by keeping CO₂ at 20% of the gas mix and changing only the composition of the other 80% of the gas. The ORP of the culture remained relatively constant between -140 and -150mV.

The culture was exposed to a mixture of 8% CO and 72% hydrogen at a flow rate of 100cc/min and CO₂ at 25cc/min for a period of 1.7 hours. Then the culture was restored to a flow of 80% hydrogen at a flow rate of 100cc/min and CO₂ at 25cc/min. Upon removal of the CO and restoration of 50% H₂, methanogenesis recovered completely within a 5 minutes (within the mixing time of the gas phase in the culture). This rapid recovery suggests that the primary effect of CO under these experimental conditions is as a reversible inhibitor of the methanogenesis process.

The culture was then exposed to a mixture of 16% CO and 64% hydrogen at a flow rate of 100cc/min and CO₂ at 25cc/min for a period of 1 hour. This higher exposure of CO showed only a 25% inhibition of methane formation rates. This suggests that the initial exposure caused an adaptation in the culture that reduced its sensitivity to CO inhibition. The culture was then restored to a flow of 80% hydrogen at a flow rate of 100cc/min and CO₂ at 25cc/min. Recovery of methanogenesis following CO removal was again immediate.

Finally, the culture was exposed to a mixture of 40% CO and 40% hydrogen at a flow rate of 100cc/min and CO₂ at 25cc/min for a period of 20 minutes. This CO exposure showed almost as much inhibition of the adapted process as occurred in the initial low level exposure of the un-adapted organisms. The culture was then restored to a flow of 80% hydrogen at a flow rate of 100cc/min and CO₂ at 25cc/min. Recovery from this level of CO was also immediate.

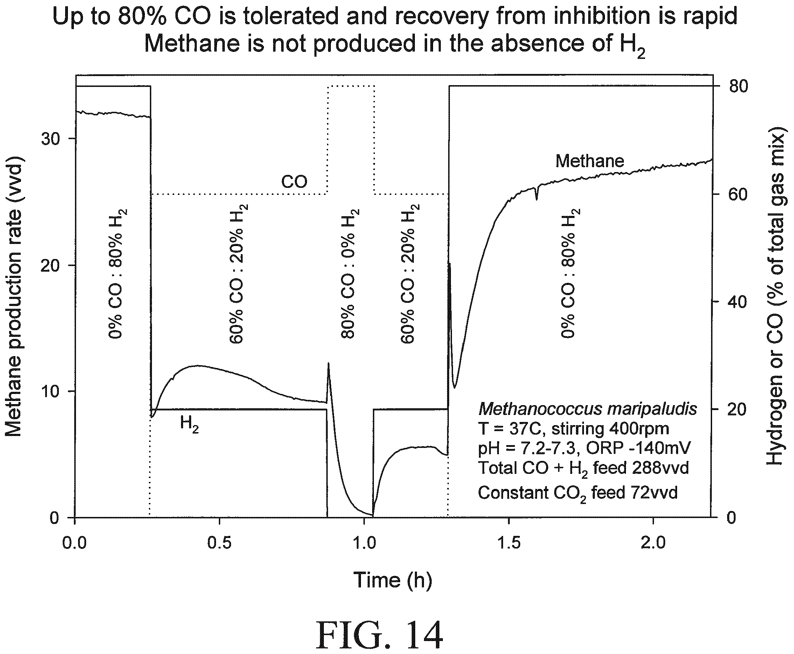

Another experiment was performed showing the effects of even higher concentration of CO on methanogenesis. A bench bioreactor containing *Methanococcus maripaludis* was prepared and maintained as set forth above. FIG. 14 shows that when the culture was given a doses of a mixture of 60% CO and 20% hydrogen at a flow rate of 100cc/min and CO₂ at 25cc/min, this led to a complete loss of methane formation in this species. Recovery from this level of CO has an immediate phase but full recovery requires several hours. These experiments show that methanogenesis achieved by methanogen cultures is tolerant to levels of CO likely to be found in industrial CO₂ streams derived from coal or biomass gasification, but that higher levels may be poorly tolerated.

While the present invention has now been described and exemplified with some specificity, those skilled in the art will appreciate the various modifications, including variations, additions, and omissions that may be made in what has been described. It is to be understood that the invention is not limited in its application to the details of construction and the arrangements of the components set forth in the previous description or illustrated in the drawings. The invention is capable of other embodiments and of being practiced or being carried out in various ways. Accordingly, it is intended that these modifications also be encompassed by the present invention and that the scope of the present invention be limited solely by the broadest interpretation that lawfully can be accorded the appended claims.

Also, it is understood that the phraseology and terminology used herein are for the purpose of description and should not be regarded as limiting. The use of "including", "having" and "comprising" and variations thereof herein is meant to encompass the items listed thereafter and equivalents thereof as well as additional items and equivalents thereof. It also is understood that any numerical value recited herein includes all values from the lower value to the upper value. For example, if a concentration range is stated as 1% to 50%, it is intended that values such as 2% to 40%, 10% to 30%, or 1% to 3%, etc., are expressly enumerated in this specification. These are only examples of what is specifically intended, and all possible combinations of numerical values between the lowest value and the highest value enumerated are to be considered to be expressly stated in this application.

Further aspects of the invention are disclosed in the following numbered paragraphs:
1. A method of converting carbon dioxide produced during an industrial process to methane comprising contacting a culture comprising methanogenic archaea with H₂ gas and an output gas from an industrial process comprising CO₂ gas in a bioreactor under suitable conditions to produce methane.
2. The method of paragraph 1 wherein the industrial process is coal gasification, biomass gasification, or liquid fuel production by biomass fermentation.
3. The method as in any one of the preceding paragraphs wherein the industrial process is ethanol production by biomass fermentation.
4. The method of paragraph 3, wherein the biomass is corn.
5. The method as in any one of the preceding paragraphs wherein the methanogenic archea comprises at least one species selected from the group consisting of *Methanobacterium alcaliphilum*, *Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arboriphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanothermobacter marburgensis, Methanothermobacter thermautotrophicum, Methanothermobacter thermoflexus, Methanothermobacter thermophilus, Methanothermobacter wolfeii, Methanothermus sociabilis, Methanocorpusculum bavaricum, Methanocorpusculum parvum, Methanoculleus chikuoensis, Methanoculleus submarinus, Methanogenium frigidum, Methanogenium liminatans, Methanogenium marinum, Methanosarcina acetivorans, Methanosarcina barkeri, Methanosarcina mazei, Methanosarcina thermophila, Methanomicrobium mobile, Methanocaldococcus jannaschii, Methanococcus aeolicus, Methanococcus maripaludis, Methanococcus vannielii, Methanococcus voltaei, Methanothermococcus thermolithotrophicus,* and *Methanopyrus kandleri.*
6. The method as in any one of the preceding paragraphs wherein the methanogenic archea is selected from the group consisting of *Methanosarcina barkeri* and *Methanococcus maripaludis.*
7. The method as in any one of the preceding paragraphs wherein the methanogenic archaea is *Methanosarcina barkeri.*
8. The method as in any one of the preceding paragraphs wherein the methanogenic archaea is *Methanococcus maripaludis.*
9. The method as in paragraphs 7 or 8 wherein the conditions include a temperature of about 35°C to about 37°C.
10. The method as in any one of the preceding paragraphs wherein the conditions include at least intermittent agitation of the culture.
11. The method as in any one of the preceding paragraphs wherein the conditions include maintaining a redox potential of about -100 mV or less.
12. The method of paragraph 11 wherein the redox potential is maintained by supplying a suitable amount of hydrogen gas.
13. The method as in any one of the preceding paragraphs, further comprising removing methane gas from the bioreactor, the removed methane gas comprising less than about 450 ppm hydrogen sulfide.
14. The method of paragraph 13, wherein the removed methane gas comprises less than about 400 ppm hydrogen sulfide.
15. The method of paragraph 13, wherein the removed methane gas comprises less than about 300 ppm hydrogen sulfide.
16. The method of paragraph 13, wherein the removed methane gas comprises less than about 200 ppm hydrogen sulfide.
17. The method of paragraph 13, wherein the removed methane gas comprises less than about 150 ppm hydrogen sulfide.
18. The method as in any one of the preceding paragraphs wherein the industrial output gas at least intermittently further comprises air.
19. The method as in any one of the preceding paragraphs wherein the industrial output gas comprises about 32% or less air by volume.
20. The method as in any one of the preceding paragraphs wherein the industrial output gas comprises from about 0.1% to about 32% air by volume.
21. The method of paragraph 18, wherein the industrial output gas comprises less than about 4% air by volume.
22. The method of paragraph 18, wherein the industrial output gas comprises at least about 4% air by volume.
23. The method as in paragraph 18, wherein the industrial output gas comprises at least about 8% air by volume.
24. The method as in paragraph 18, wherein the industrial output gas comprises at least about 16% air by volume.
25. The method as in any one of the preceding paragraphs wherein the industrial output gas further comprises carbon monoxide.
26. The method as in any one of the preceding paragraphs wherein the industrial output gas comprises less than about 40% carbon monoxide by volume.
27. The method as in any one of the preceding paragraphs wherein the industrial output gas comprises less than about 8% carbon monoxide by volume.
28. The method of paragraph 25, wherein the industrial output gas comprises at least about 8% carbon monoxide by volume.
29. The method of paragraph 25, wherein the industrial output gas comprises at least about 16% carbon monoxide by volume.
30. The method as in any one of the preceding paragraphs wherein pressure within the bioreactor is from about 0.5 atmospheres to about 500 atmospheres.
31. A bioreactor comprising:
   a culture of methanogenic archaea;
   a source of an output gas from an industrial process comprising CO₂ that feeds into the bioreactor;
   a source of hydrogen gas that feeds into the bioreactor;
   a gas feed from the bioreactor for removing gas from the bioreactor;
   a feed for providing fresh medium; and
   a feed for removing the culture.
32. The bioreactor of paragraph 31 wherein the methanogenic archea comprises one or more species selected from the group consisting of *Methanobacterium alcaliphilum*, *Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arboriphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanothermobacter marburgensis, Methanothermobacter thermautotrophicum, Methanothermobacter thermoflexus, Methanothermobacter thermophilus, Methanothermobacter wolfeii, Methanothermus sociabilis, Methanocorpusculum bavaricum, Methanocorpusculum parvum, Methanoculleus chikuoensis, Methanoculleus submarinus, Methanogenium frigidum, Methanogenium liminatans, Methanogenium marinum, Methanosarcina acetivorans, Methanosarcina barkeri, Methanosarcina mazei, Methanosarcina thermophila, Methanomicrobium mobile, Methanocaldococcus jannaschii, Methanococcus aeolicus, Methanococcus maripaludis, Methanococcus vannielii, Methanococcus voltaei, Methanothermococcus thermolithotrophicus,* and *Methanopyrus kandleri.*
33. The bioreactor as in any one of paragraphs 31-32 wherein the industrial output gas further comprises air.
34. The bioreactor as in any one of paragraphs 31-33 wherein the industrial output gas further comprises carbon monoxide.

## Claims

1. A bioreactor comprising:
a) a culture of hydrogenotrophic methanogenic archaea;
b) a source of an output gas from an industrial process comprising CO₂ that feeds into the bioreactor wherein the output gas comprises about 32% or less air by volume; and
c) a source of hydrogen gas that feeds into the bioreactor, wherein the redox potential in the bioreactor is maintained at -100 mV or less with the hydrogen gas;
wherein the hydrogenotrophic methanogenic archaea converts the hydrogen gas and the CO₂ gas to methane.

2. The bioreactor of claim 1, further comprising a gas feed from the bioreactor for removing gas from the bioreactor.

3. The bioreactor of claim 1 or 2, further comprising a feed for providing fresh medium.

4. The bioreactor of any one of claims 1-3, further comprising a feed for removing the culture.

5. The bioreactor of any one of claims 1-4, wherein the methane gas removed from the bioreactor comprises less than 450 ppm, less than 400 ppm, less than 300 ppm, less than 200 ppm, or less than 150 ppm hydrogen sulfide.

6. The bioreactor of any one of claims 1-5, wherein the hydrogenotrophic methanogenic archaea is of the *Methanococci* class, *Methanomicrobia* class, or the *Methanobacteria* class.

7. The bioreactor of any one of claims 1-6, wherein the culture is a pure culture of a microorganism of the *Methanococci* class, *Methanomicrobia* class, or the *Methanobacteria* class.

8. The bioreactor of claim 6 or 7, wherein the microorganism of the *Methanococci* class is a *Methanococcus maripaludis.*

9. The bioreactor of claim 6 or 7, wherein the microorganism of the *Methanobacteria* class is a *Methanothermobacter thermautotrophicum.*

10. The bioreactor of claim 6 or 7, wherein the microorganism of the *Methanomicrobia* class is a *Methanosarcina barkeri.*

11. The bioreactor of any one of claims 1-10, wherein the industrial output gas comprises at least 4% air by volume or at least 8% air by volume.

12. The bioreactor of any one of claims 1-11, wherein the methane gas removed from the bioreactor comprises less than 450 ppm hydrogen sulfide.

13. The bioreactor of any one of claims 1-12, wherein the culture of hydrogenotrophic methanogenic archaea is prepared in a second reactor vessel, wherein fresh medium is supplied to the bioreactor and second reactor vessels through a first medium feed line attached to the bioreactor, and a second medium feed line attached to the second reactor vessel, and transferring by a first gas feed line at least a portion of gas in the bioreactor vessel to the second bioreactor vessel, wherein the hydrogenotrophic methanogenic archaea culture in the second reactor converts hydrogen gas and CO₂ gas to produce methane.

14. The bioreactor of any one of claims 1-12, wherein the bioreactor is a stratified bioreactor, optionally, comprising a mechanical impellar at the top of the bioreactor which mechanical impellar moves a mixing apparatus within the bioreactor, wherein the carbon dioxide, hydrogen and nutrients enter at the bottom of the bioreactor, and wherein the amount of carbon dioxide decreases from the bottom of the bioreactor to the top of the bioreactor.

15. The bioreactor of any one of claims 1-12, wherein the bioreactor is a cascaded bioreactor, optionally, comprising a first compartment, a second compartment, and a third compartment, wherein the carbon dioxide, hydrogen and cell nutrients enter at the bottom of the first compartment, wherein gas produced in the first compartment is transferred from the top of the first compartment to the bottom of the second compartment along with cell nutrients, wherein gas produced in the second compartment is transferred from the top of the second compartment to the bottom of the third compartment along with cell nutrients.

16. The bioreactor of any one of claims 1-15, wherein the industrial output gas at least intermittently further comprises carbon monoxide.

17. The bioreactor of any one of claims 1-16, wherein the culture is at least intermittently agitated.
